# EUROPÄISCHE PATENTANMELDUNG

(11) **EP 4 556 944 A1**
(43) Veröffentlichungstag der Anmeldung: **21.05.2025**
(21) Anmeldenummer: 23210860.5
(22) Anmeldetag: 20.11.2023
(51) Int. Cl.: G01S 7/41, A61B 5/0507, A61B 5/08, A61B 5/113, G01S 13/42, G01S 13/88

(54) **COMPUTERIMPLEMENTIERTES VERFAHREN ZUR MESSUNG UND ÜBERWACHUNG VON VITALPARAMETERN SOWIE ERKENNUNG VON NOTFALL- UND STURZSITUATIONEN IN EINEM ÜBERWACHUNGSBEREICH UNTER ANWENDUNG EINER SENSORANORDNUNG**

(71) Anmelder: VmedD GmbH, 01189 Dresden (DE)
(72) Erfinder: SOCH, Jeremias, 01705 Freital (DE)
(74) Vertreter: Lippert Stachow Patentanwälte Rechtsanwälte Partnerschaft mbB

(57) **Zusammenfassung**

Die Erfindung betrifft ein computerimplementiertes Verfahren zur Messung und Überwachung von Vitalparametern sowie zur Erkennung von Notfall- und Sturzsituationen in einem Überwachungsbereich unter Anwendung einer Sensoranordnung. Die Aufgabe sowohl stationäre als auch häusliche Bereiche fortlaufend zu überwachen und die aufgenommenen Daten effizient und effektiv auszuwerten, um im Notfall sofort Hilfe zu rufen oder auch Krankheitsbilder zu erkennen, wird dadurch gelöst, dass empfangene Reflexionssignale von ausgesendeten Radarsignalen derart aufbereitet und ausgewertet werden, dass für eine Anwesenheitserkennung eines Objektes eine X-/Y-Position im Überwachungsbereich detektiert und überwacht wird, und für eine Sturzerkennung des Objektes zusätzlich zu einer X-/Y-Position im Überwachungsbereich sowohl eine räumliche als auch zeitliche Überwachung einer Z-Position des Objektes detektiert und überwacht wird, und für eine Messung von Vitalparametern des Objektes Phasenwerte der ermittelten Reflexionssignale für dieses Objekt ausgewertet und mit den Werten der Anwesenheits- und Sturzerkennung des Objektes verknüpft werden.

## Beschreibung

Die Erfindung betrifft ein computerimplementiertes Verfahren zur Messung und Überwachung von Vitalparametern sowie zur Erkennung von Notfall- und Sturzsituationen in einem Überwachungsbereich unter Anwendung einer Sensoranordnung.

Die laufende Beurteilung von Gesundheit und Wohlbefinden, insbesondere zu Hause, kann ein wirksames Mittel sein, um frühe Anzeichen für den Beginn von Beschwerden zu erkennen, sodass eine frühzeitige medizinische Intervention angeboten werden kann, wodurch die Notwendigkeit einer Krankenhauseinweisung reduziert und in vielen Fällen ganz vermieden wird.

Auch im Hinblick auf den steigenden Pflegenotstand aufgrund fehlenden Personals im Gesundheitswesen, dem darauf beruhenden Bettenmangel im stationären Bereich aber auch die immer weiter steigende Lebenserwartung der Menschen und damit die Alterung der Bevölkerung lässt die Notwendigkeit für eine laufende Überwachung sowohl im stationären als auch häuslichen Bereich immer notwendiger erscheinen.

Eine fortlaufende Überwachung verschiedener Gesundheitsparameter, die den Gesundheitszustand anzeigen, können dazu dienen, frühzeitig Krankheiten zu erkennen bzw. eine vernünftige Risikoabschätzung für eine Person zu treffen, die z. B. eine chronische Herzinsuffizienz oder dergleichen entwickelt.

Auch eine fortlaufende Überwachung von älteren Menschen in ihrer gewohnten häuslichen Umgebung kann diese unterstützen, länger selbstständig zu bleiben, da im Notfall sofort Angehörige oder der Rettungsdienst informiert werden können, um der Person zu helfen. Gerade wenn ältere Menschen allein leben und z. B. in ihrer Wohnung stürzen, sind sie oftmals nicht in der Lage selbst Hilfe zu rufen und werden möglicherweise erst Tage später gefunden.

Bisherige Systeme für die Überwachung von Innenräumen erfordern die Verwendung einer großen Anzahl von Erfassungseinheiten, Sende- und Empfangseinheiten und zusätzliche Geräte, wodurch die Kosten sehr hoch sind.

Aus der US 2021/141082 A1 sind ein System und Verfahren zum Erzeugen und/oder Aktualisieren einer Innenraumumgebungskarte bekannt, wobei eine Innenraumumgebung mittels einem oder mehrerer Sensoren erfasst wird und aus den Verarbeitungsdaten der Umgebung die Innenraumumgebungskarte erzeugt und aktualisiert wird. Zur Erfassung der Innenraumumgebung wird eine 3D-Radartechnologie verwendet. Funkwellen sind in der Lage, Objekte bei schlechten Sichtverhältnissen wie Dunkelheit, Rauch, Dunst und Nebel zu erkennen. Mit dem System aus der US 2021/141082 A1 kann eine dreidimensionale (3D) Voxel-Karte der Umgebung erstellt und in Echtzeit aktualisiert werden. Jedem Voxel wird dabei ein Zeitstempel und optional jeweilige Ereignismerkmale zugeordnet.

Der nachfolgend beschriebenen Erfindung liegt die Aufgabe zugrunde, eine Möglichkeit zu bieten, sowohl stationäre als auch häusliche Bereiche fortlaufend zu überwachen und die aufgenommenen Daten effizient und effektiv auszuwerten, um im Notfall sofort Hilfe zu rufen oder auch Krankheitsbilder zu erkennen, so dass diesen Personen möglichst frühzeitig geholfen werden kann.

Es ist auch eine Aufgabe der vorliegenden Erfindung ein Verfahren und eine Vorrichtung zur Verfügung zu stellen, welche einfach auf verschiedene Anwendungsfälle adaptiert werden können.

Diese Aufgabe wird durch ein computerimplementiertes Verfahren zur Messung und Überwachung von Vitalparametern sowie zur Erkennung von Notfall- und Sturzsituationen in einem Überwachungsbereich gemäß dem unabhängigen Anspruch 1 gelöst. Das erfindungsgemäße Verfahren läuft auf einem Mikroprozessor und umfasst folgende Schritte:
- Empfangen von Daten, die mittels einer Sende- und Empfangseinheit erfasst werden, wobei die Sende- und Empfangseinheit ausgebildet ist Radarsignale in den Überwachungsbereich auszusenden und ihre Reflexionssignale aus dem Überwachungsbereich zu empfangen,
- Berechnen von Entfernungsdaten aus den empfangenen Reflexionssignalen,
- Berechnen einer Reflexionsstärke für jede dreidimensionale (3D-) Position im Überwachungsbereich aus den empfangenen Reflexionssignalen,
- Ermitteln gültiger Reflexionswerte aus der Reflexionsstärke für jede 3D-Position im Überwachungsbereich und Erstellen einer Punktwolke,
- Gruppieren einzelner Punkte der Punktwolke zu einem Objekt und Verfolgen einer Position des Objektes im Überwachungsbereich, wobei
- für eine Anwesenheitserkennung eines Objektes eine X-/Y-Position im Überwachungsbereich detektiert und überwacht wird, und
- für eine Sturzerkennung des Objektes zusätzlich zu einer X-/Y-Position im Überwachungsbereich sowohl eine räumliche als auch zeitliche Überwachung einer Z-Position des Objektes detektiert und überwacht wird, und
- für eine Messung von Vitalparametern des Objektes Phasenwerte der ermittelten Reflexionssignale für dieses Objekt ausgewertet und mit den Werten der Anwesenheits- und Sturzerkennung des Objektes verknüpft werden.

In einem ersten Schritt sendet die Sendeeinheit einer Sensoranordnung über ein phasengesteuertes Antennen-Array Radarsignale im 60-64 GHz-Bereich in einen Überwachungsbereich aus. Weitere Frequenzbereiche, z. B. 120 GHz, sind ebenfalls realisierbar. Das phasengesteuerte Antennen-Array besteht aus mindestens 3 Sendeantennen und einer Empfangsantenne, es kann aber auch mehr als eine Empfangsantenne verwendet werden. Die Reflexionssignale, die auf die Sensoranordnung zurückreflektiert werden, werden von einer Empfangseinheit der Sensoranordnung empfangen. Dabei wird ein In-Phase- und Quadrature-Verfahren vor der Digitalisierung der Signale eingesetzt, um sowohl Amplituden- als auch Phasenwerte zu erfassen, die dann ausgewertet werden. Zur Erhöhung des Signal-Rauschverhältnisses und der damit besseren/exakteren/genaueren Erfassung der Amplituden- und Phasenwerte werden wiederholt Radarsignale ausgesandt und empfangen, bevor die empfangenen Daten für einen Verarbeitungszyklus ausgewertet werden.

Aus den empfangenen Reflexionssignalen werden Entfernungsdaten berechnet. Dafür wird der Überwachungsbereich vom Sensor ausgehend radial in Entfernungsabschnitte eingeteilt. Die Ausdehnung eines Entfernungsabschnittes beträgt wenige Zentimeter, vorteilhaft sind 3cm. Dies ist insbesondere abhängig von der Bandbreite der eingesetzten Sendeeinheit. Durch eine Frequenzanalyse der Reflexionssignale wird für jeden Entfernungsabschnitt ein Amplituden- und Phasenwert berechnet. Der Amplitudenwert ist eine Maß für die Reflexionsstärke der von den Objekten in dem Entfernungsabschnitt reflektierten Signale. Der Phasenwert ist ein Maß für die Entfernung eines Objektes innerhalb eines Entfernungsabschnittes. Jedoch stellt der Phasenwert keinen absoluten Wert für eine Entfernung dar, da aufgrund der kleinen Wellenlänge der Radarsignale im Gegensatz zum größeren Entfernungsabschnitt, mehrere Entfernungen dem gleichen Phasenwert entsprechen können. Es können daher nur Abstandsänderungen gemessen/detektiert werden. Bewegt sich demnach ein Objekt innerhalb eines Entfernungsabschnittes können die Änderungen mit einer Genauigkeit im Mikrometerbereich erfasst werden, beispielsweise mit einer Genauigkeit von 30µm.

Vorteilteilhaft ist, wenn aus den empfangenen Reflexionssignalen statische Objekte herausgerechnet werden, da diese sich nicht bewegen und für eine Sturzdetektion oder Vitalparameterbestimmung keine Rolle spielen. Damit lässt sich das Verfahren vereinfachen und beschleunigen. Insbesondere ist es daher vorteilhaft, wenn die empfangenen Reflexionssignale vorkonfiguriert werden, so dass aus den Reflexionssignalen Objekte mit verschiedenen Bewegungsintensitäten unterschieden werden können. Dafür werden die Daten der empfangenen Reflexionssignale mit verschiedenen Berechnungsvorschriften bearbeitet, die resultierenden Daten werden abgespeichert und anschließend weiterverarbeitet. So lassen sich die Ergebnisse später kombinieren. Die Vorkonfiguration der Daten kann beispielsweise so aussehen, dass mittels einer ersten Berechnungsvorschrift statische Objekte aus den Daten herausgerechnet werden. Statische Objekte sind Objekte, die keinerlei Bewegungen aufweisen. Dadurch können beispielsweise Lebewesen besser von anderen Objekten unterschieden werden. Die daraus resultierenden Daten vereinfachen und beschleunigen die Erkennung von Stürzen und die Bestimmung von Vitalparametern. Mittels einer zweiten Berechnungsvorschrift werden Bewegungen von Objekten herausgerechnet, um statische Objekte im Raum zu erkennen. Dies ermöglicht eine Kartierung des überwachten Raumes und ist z. B. hilfreich für die Anwesenheitserkennung eines Objektes/Person im Bett und/oder im Raum. Mittels einer weiteren Berechnungsvorschrift werden die resultierenden Daten nach der ersten Berechnungsvorschrift über mehrere Verarbeitungszyklen miteinander neu kombiniert. Dadurch werden schnelle große Bewegungen, z. B. das Gehen oder das Fallen von Lebewesen, falls vorhanden, herausgerechnet und feine langsame Bewegungen, wie z. B. beim Atmen und Sprechen, werden verstärkt. Dies ist hilfreich, um Lebewesen zu erkennen, wenn sie sich sehr ruhig verhalten, z. B. während des Schlafens oder nach einem Sturz.

Die Bearbeitung der Daten erfolgt getrennt für jeden Entfernungsabschnitt, der auch als Entfernungsbereich bezeichnet wird.

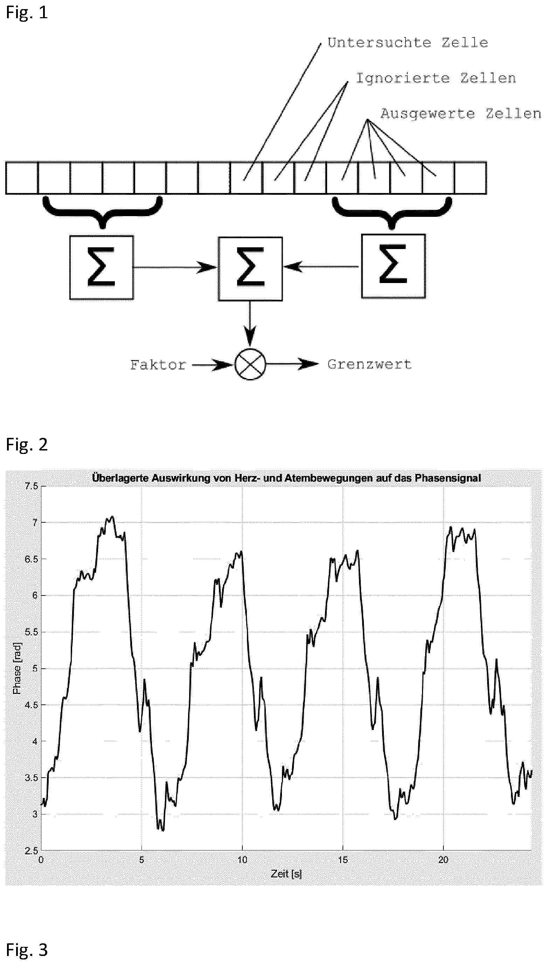

In einem weiteren Schritt wird für jede Position im dreidimensionalen Raum des Überwachungsbereiches eine Reflexionsstärke aus den empfangenen Reflexionssignalen berechnet. Ausgehend von einem Kugelkoordinatensystem wird jeder Entfernungsbereich zusätzlich in Winkelbereiche für den Azimut-Winkel und Elevationswinkel eingeteilt. Mittels eines Spektrum-basierten Beamforming-Verfahrens wird für diese durch Entfernung und Raumwinkel definierten Raumbereiche, jeweils die Reflexionsstärke berechnet. Aus der Reflexionsstärke für jede 3D-Position im Überwachungsbereich werden gültige Reflexionswerte ermittelt und daraus eine Punktwolke erstellt. Unter einem gültigen Reflexionswert wird eine Signalstärke der empfangenen Reflexionssignale verstanden, die von einem Zielobjekt stammt. Diese können sehr unterschiedlich sein, da sie z. B. vom Abstand und dem Oberflächenmaterial des Objektes abhängig sind. Um dieses Problem zu umgehen, wird der Grenzwert für einen gültigen Reflexionswert für jeden Raumbereich, welcher auch als Zelle bezeichnet wird, aus seiner lokalen Umgebung neu berechnet. Dafür wird jede Zelle mit benachbarten Zellen verglichen. Dazu wird die Summe der umliegenden Zellen für einen bestimmten Bereich ermittelt. Dabei können direkt anliegende Zellen ignoriert werden. Die Summe wird mit einem festen Faktor zu einem Grenzwert verrechnet. Der Grenzwert wird mit dem Wert der zu untersuchenden Zelle verglichen. Ist der Wert der Zelle größer als der Grenzwert, handelt es sich um einen gültigen Reflexionswert und der Raumbereich wird als Punkt in die Punktwolke übernommen. Empirisch wird der Faktor auf ein Optimum eingestellt, so dass die Erkennung aller Zielobjekte bei minimaler Anzahl an Fehldetektionen erfolgt. Die Auswertung der Zellen kann sowohl in Kombination aus 1D- und 2D-Verabeitung, welche genau genug und schnell ist, als auch direkt als 3D-Verabeitung, erfolgen. Für jeden übernommenen Punkt werden die Position, das Signal-Rausch-Verhältnis und die relative Geschwindigkeit zum Sensor berechnet und abgespeichert. Dies ist schematisch in Fig. 1 dargestellt.

In einem weiteren Schritt werden einzelne Punkte der Punktwolke, die ähnliche Werte bezüglich Position, Geschwindigkeit und Signal-Rausch-Verhältnis haben, zu einem Objekt gruppiert. Dabei können Punkte entweder einem aus vorherigen Verarbeitungszyklus bekannten Objekt zugeordnet werden, oder Punkte können zu einem neuen Objekt zusammengefasst werden. Punkte, die nicht zugeordnet werden können, werden verworfen.

Zunächst wird geprüft, welche Punkte sich zu einem vorhandenen Objekt zuordnen lassen. Dies umfasst zwei Schritte. Erstens die Berechnung eines Grenzwertes für die Entfernung und zweitens die Berechnung einer Punktezahl für die Zugehörigkeit. Bei der Berechnung des Entfernungsgrenzwertes werden der prognostizierte Zustandsvektor (Ort, Geschwindigkeit, Beschleunigung) des Objektes, die vorherige Verteilung von Punkten dieses Objektes im Raum und die Messunsicherheit mit einbezogen. Hat ein Punkt der Punktwolke einen kleineren Abstand als der berechnete Grenzwert, so kann er zu diesem Objekt gehören. Dies kann für mehrere Objekte erfüllt sein. Für eine eindeutige Zuordnung wird im nächsten Schritt die Punktezahl für die Zugehörigkeit berechnet. Dazu wird der Mahalanobis-Abstand, mit verstärkter Faktorisierung der Geschwindigkeit, zwischen einem Punkt und jedem möglichen Objekt berechnet. Da die zu unterscheidenden Objekte schon räumlich nah beieinanderliegen, liefert die Verstärkung der Geschwindigkeit während der Berechnung eine bessere Zuordnung. Der Punkt wird dem Objekt mit der höchsten Punktezahl zugeordnet.

Werden Punkte zu einem neuen Objekt zusammengefasst, werden die Werte eines beliebigen Punkts als Objektzentrum und Objektgeschwindigkeit angenommen. Nacheinander wird für alle restlichen Punkte überprüft, ob sie die Grenzwerte für Abstand und Geschwindigkeitsunterschied einhalten. Sind die Grenzen erfüllt, wird der neue Punkt dem Objekt hinzugefügt und das Objektzentrum und die Objektgeschwindigkeit werden neu berechnet, um mit dem nächsten Punkt erneut den Vergleich zu starten. Nachdem alle vorhandenen Punkte getestet wurden, wird das erstellte Objekt auf Gültigkeit geprüft. Dabei müssen die Grenzwerte für die Anzahl der Punkte, das kombinierte Signal-Rausch-Verhältnis sowie die minimale Geschwindigkeit überschritten werden, damit ein gültiges neues Objekt abgespeichert wird.

Allgemein wird die Position des gruppierten Objektes im Überwachungsbereich verfolgt, so dass für eine Anwesenheitserkennung eines Objektes eine X-/Y-Position im Überwachungsbereich detektiert und überwacht werden kann.

Des Weiteren wird für eine Sturzerkennung des Objektes zusätzlich zu der X-/Y-Position im Überwachungsbereich sowohl eine räumliche als auch zeitliche Überwachung einer Z-Position des Objektes detektiert und überwacht. Dabei ist der Schwerpunkt der Punktwolke aller Punkte eines Objektes die verwendete Z-Position. Es können aber auch die minimale/maximale Z-Position (niedrigster/höchster Punkt) mit in die Bewertung einbezogen werden. Beispielsweise schwankt beim Gehen die Z-Position erheblich, da unterschiedliche Körperteile in jedem Verarbeitungszyklus verschieden stark reflektieren. Liegt die Person/das Objekt aber am Boden, ist egal wo Reflexionen auftreten, der Schwerpunkt wird immer auf einer niedrigen Höhe sein. Zur Verifizierung und Feinauswertung der aufgenommenen Daten erfolgt erfindungsgemäß noch eine Messung von Vitalparametern des Objektes, wobei dafür Phasenwerte der aufgenommenen Reflexionssignale dieses Objektes ausgewertet und mit den Werten der Anwesenheits- und Sturzerkennung des Objektes verknüpft werden.

Die Phasenwerte stellen Objektbewegungen im Millimeter- und Mikrometerbereich dar. Beim Menschen reflektiert die Haut die Radarsignale. Eine Bewegung der Haut, verursacht durch Atmung und Herzschlag, bewirkt eine Änderung der Phase. Die Änderung der Phase ist direkt proportional zur Abstandsänderung verursacht durch die Hautbewegung (Fig. 2).

In einer Ausgestaltung des erfindungsgemäßen computerimplementierten Verfahrens werden mittels einer Wärmesignatur an einer Objektposition, die mittels eines Thermopile-Arrays erfasst wird, Rückschlüsse auf die Präsenz des Objektes gezogen. An einer Objektposition befindet sich ein Objekt, das verifiziert werden soll. Erfasst das Thermopile-Array eine Wärmestrahlung, die höher als die Umgebung des Objektes ist, so lässt sich daraus ableiten, dass das Objekt an dieser Position möglicherweise ein Lebewesen, z. B. ein Mensch oder ein Tier sein kann. Diese Annahme muss jedoch noch weiter spezifiziert werden.

In einer weiteren Ausgestaltung des erfindungsgemäßen Verfahrens wird daher mittels einer Feinbewegungsprüfung auf Basis von Phasenwerten detektiert, ob sich das erfasste Objekt bewegt, wobei daraus eine gesicherte Aussage getroffen werden kann, ob es sich um ein Lebewesen handelt oder nicht. Die Phasenwerte werden aus den empfangenen Reflexionssignalen für jede 3D-Position im Überwachungsbereich extrahiert und mit dem gruppierten Objekt verknüpft.

In einer anderen Ausgestaltung des erfindungsgemäßen Verfahrens umfasst die Feinbewegungsprüfung zur Bestimmung von Vitalparametern die folgenden Schritte, wobei damit insbesondere eine Atemfrequenz des Objektes bestimmt werden kann:
- Auswählen einer Objektposition und der dazugehörigen Reflexionssignale sowie Phasenwerte in einem Raumbereich,
- Filtern der Reflexionssignale mit einem IIR-Bandpassfilter für den Atemfrequenzbereich,
- Durchführen einer Frequenzanalyse mittels Fourier-Transformation und Berechnen eines Amplitudenspektrums für den Atemfrequenzbereich,
- Ermitteln der höchsten Amplitudenwerte des Amplitudenspektrums sowie deren entsprechende Frequenzwerte und Grundrauschen,
- Sortieren der ermittelten Amplitudenwerte nach ihrem Signal-Rausch-Verhältnis, wobei ein vorbestimmter Prozentsatz der ermittelten Amplitudenwerte zur Weiterverarbeitung ausgewählt und verwendet wird,
- Berechnen eines Medianwertes aller Frequenzwerte der ausgewählten und verwendeten Amplitudenwerte, wobei der Medianwert einen Wert für die Atemfrequenz darstellt.

In einer weiteren Ausgestaltung des erfindungsgemäßen Verfahrens umfasst die Feinbewegungsprüfung zur Bestimmung von Vitalparametern die folgenden Schritte, wobei damit insbesondere eine Herzschlagrate des Objektes erfasst werden kann:
- Auswählen einer Objektposition und der dazugehörigen Reflexionssignale sowie Phasenwerte in einem Raumbereich,
- Filtern der Reflexionssignale mit einem IIR-Bandpassfilter für den Frequenzbereich der Herzschlagrate,
- Durchführen einer Frequenzanalyse mittels Fourier-Transformation und Berechnen eines Amplitudenspektrums für den Frequenzbereich der Herzschlagrate,
- Bereinigen des berechneten Amplitudenspektrums von Störungen durch harmonische Frequenzen der Atemfrequenz und zufälliger Objektbewegungen, indem harmonische Frequenzen der potentiellen Herzschlagrate detektiert und ein Fundamentalfrequenzwert in einem Datenpuffer gewichtet gespeichert wird,
- Durchführen einer Clusteranalyse auf die in dem Datenpuffer gespeicherten Frequenzen, wobei ein Frequenzmittelwert des größten Clusters gespeichert wird,
- Wiederholen des vorbenannten Verarbeitungsschrittes und Berechnen eines Medianwertes der über mehreren Verarbeitungszyklen gespeicherten Frequenzmittelwerte, wobei der Medianwert einen Wert für die Herzschlagrate darstellt.

Körperfunktionen, wie die Atemfrequenz oder die Herzschlagrate weisen eine Periodizität in verschiedenen Frequenzbereichen auf, die für die Bestimmung dieser Vitalparameter in vorteilhafterweise erfindungsgemäß genutzt wird.

Mit Hilfe der Objektposition werden die richtigen Entfernungsabschnitte mit ihren Phasenwerten ausgewählt.

Die Phasenwerte werden in aufeinanderfolgenden Verarbeitungszyklen abgespeichert. Für jedes Paar aus Sende- und Empfangsantenne stehen in jedem Entfernungsabschnitt Phasenwerte zur Verfügung. Zunächst werden diese Signale mit einem IIR-Bandpassfilter gefiltert. Es werden zwei verschiedene Filter eingesetzt, jeweils einer für den Frequenzbereich der Atmung und einer für den Frequenzbereich des Herzschlags zur Bestimmung der Herzschlagrate. Die Grenzfrequenzen der Filter sind so gewählt, dass typische Werte für Herz- und Atemfrequenz nicht gefiltert werden. Um eine Frequenzanalyse der gefilterten Daten zu erstellen, wird anschließend eine diskrete Fourier-Transformation auf die Daten angewendet. Das Ergebnis der Fourier-Transformation wird genutzt, um das Amplitudenspektrum für die entsprechenden Frequenzbereiche zu berechnen. Für die Auswertung werden die höchsten Amplitudenwerte und ihr entsprechender Frequenzwert sowie das Grundrauschen ermittelt. Für alle Antennenpaare in den gewählten Entfernungsabschnitten werden diese Werte ermittelt und miteinander verglichen.

Für die Atmung werden alle Amplitudenwerte nach ihrem Signal-Rausch-Verhältnis sortiert. Ausgehend von dem höchsten Signal-Rausch-Verhältnis wird nur ein gewisser Prozentsatz für die Weiterverarbeitung verwendet. Aus allen Frequenzwerten dieser gewählten Amplitudenwerte wird der Median berechnet. Über mehrere Verarbeitungsschritte werden diese Werte gespeichert und in jedem Zyklus der Medianwert berechnet. Dieser Wert ist das Ergebnis für die Atemfrequenz.

Bei der Auswertung des Spektrums für die Herzschlagrate müssen Störungen durch harmonische Frequenzen der Atmung und durch zufällige Körperbewegungen beachtet werden. Diese verursachen ebenfalls hohe Amplituden im Frequenzbereich der Herzschlagrate. Als ein zuverlässiges Unterscheidungsmerkmal hat sich das Vorhandensein von hohen Amplitudenwerten bei den harmonischen Frequenzen gezeigt. Eine harmonische Frequenz gilt als vorhanden, wenn in einem Frequenzbereich um das ganzzahlige Vielfache der Grundfrequenz eines Amplitudenwertes ein Grenzwert für das Signal-Rausch-Verhältnis überschritten wird. Störungen haben auch hohe Amplitudenwerte im Herzfrequenzbereich (0,8 Hz - 2,0 Hz), aber keine hohen Werte im Bereich der harmonischen (1,6 Hz - 6,0 Hz). Nur wenn keine harmonischen Frequenzen gefunden werden, dann darf der Frequenzwert, der in den Datenpuffer übernommen wird, kein Vielfaches der Atemfrequenz sein. Bei der Bestimmung der Herzschlagrate muss daher das berechnete Amplitudenspektrum von diesen störenden harmonischen Frequenzen der Atmung bereinigt werden.

In einer anderen weiteren Ausgestaltung des erfindungsgemäßen Verfahrens werden daher bei der Bereinigung des berechneten Amplitudenspektrums nur Frequenzwerte in den Datenpuffer übernommen werden, die folgende Regeln erfüllen:
- Ist eine 1. und 2. Harmonische des ausgewählten Frequenzwertes vorhanden, wird der Frequenzwert viermal in den Datenpuffer übernommen,
- Ist die 1. oder 2. Harmonische des ausgewählten Frequenzwertes vorhanden, wird der Frequenzwert dreimal in den Datenpuffer übernommen,
- Ist der Amplitudenwert der höchste im Amplitudenspektrum und ist der zugehörige Frequenzwert keine Harmonische der Atemfrequenz wird der Frequenzwert zweimal übernommen,
- Ist eine Differenz zwischen einem Frequenzwert zu einem der drei höchsten Amplitudenwerte im Amplitudenspektrum und einem Ergebnis für die Herzschlagrate aus einem vorherigen Verarbeitungszyklus kleiner als ein Grenzwert, wird dieser Frequenzwert übernommen, wobei
- die Regeln in der vorbenannten Reihenfolge überprüft werden bis eine Regel zutrifft.

Alle gefundenen Frequenzwerte aller Antennenpaare aus allen Entfernungsabschnitten werden überprüft und in den Datenpuffer übernommen. Dabei gelten bestimmte Regeln, ob und wie der Wert übernommen wird:
- ist die 1. und 2. Harmonische des ausgewählten Frequenzwertes vorhanden, wird der Frequenzwert viermal übernommen,
- ist die 1. oder 2. Harmonische des ausgewählten Frequenzwertes vorhanden, wird der Frequenzwert dreimal übernommen,
- ist der Amplitudenwert der höchste im Amplitudenspektrum und ist der Frequenzwert keine Harmonische der Atemfrequenz wird der Wert zweimal übernommen,
- ist die Differenz zwischen dem Frequenzwert zu einem der drei höchsten Amplitudenwerte im Amplitudenspektrum und dem Ergebnis für die Herzschlagrate aus einem vorherigen Verarbeitungszyklus kleiner als ein Grenzwert, wird der Wert einfach übernommen.

Die Regeln werden in der genannten Reihenfolge überprüft und sobald für ein Antennenpaar für einen Frequenzwert eine der Regeln zutrifft, werden keine weiteren Regeln und Frequenzwerte für dieses Antennenpaar mehr überprüft.

Auf den Datenpuffer mit allen gesammelten Frequenzwerten wird eine Clusteranalyse angewendet. Das Clustering ist vorteilhaft, um Ausreißer von der Mittelung auszuschließen und Störungen in einzelnen Entfernungsabschnitten zu unterdrücken. Der Mittelwert des größten Clusters wird verwendet. Über mehrere Verarbeitungsschritte werden diese Werte gespeichert und anschließend der Medianwert berechnet. Dieser Wert ist das Ergebnis für die Herzschlagrate.

In einer Ausgestaltung des erfindungsgemäßen Verfahrens wird ein Vertrauenslevel definiert, welches genutzt wird, um zu prüfen, ob berechnete Werte der Vitalparameter plausibel sind oder ob es sich um falsch positiv detektierte Vitalparameter handelt.

Es hat sich als vorteilhaft erwiesen, ein sogenanntes Vertrauenslevel zu definieren, welches genutzt wird, um zu prüfen, ob berechnete Werte plausibel sind oder ob es sich um falsch positiv detektierte Werte handelt, die dann verworfen werden müssen. Das Ergebnis der Herzschlagrate wird in jedem Zeitschritt abgespeichert. Anschließend wird über einen bestimmten Zeitraum der Median dieser Werte berechnet. Anschließend wird gezählt wie viele Werte in diesem Zeitraum eine Differenz zum Median haben, die kleiner als ein definierter Grenzwert ist. Das Verhältnis dieser Anzahl zu der Gesamtanzahl an Werten definiert das Vertrauenslevel.

Hat der Algorithmus in letzter Zeit viele unterschiedliche Werte ermittelt, ist das Vertrauen niedrig, ansonsten ist es hoch. Das hat den Vorteil, dass Werte aus Zeiträumen in denen der Sensor wahrscheinlich falsch positioniert ist, nicht für die Mittelung der Minuten-, Stunden- oder Tageswerte mit einbezogen werden.

In einer weiteren Ausgestaltung des erfindungsgemäßen Verfahrens wird der Überwachungsbereich konfiguriert, indem Objekte im Überwachungsbereich mittels einer Objektklassifikation klassifiziert werden und die Daten der Objektklassifikation für einen selbstlernenden Algorithmus für das Erstellen und kontinuierliche Verbessern einer Raumkonfiguration des Überwachungsbereiches genutzt wird.

Für die Raumkonfiguration werden Daten gesammelt und dem Algorithmus zum Lernen zur Verfügung gestellt. Je mehr Daten dem Algorithmus mit der Zeit zur Verfügung stehen, desto genauer und zuverlässiger kann die Raumkonfiguration erfolgen. Zum Beispiel wird die Position eines Bettes selbstlernend vom Sensor dadurch ermittelt, dass ein Mensch immer wieder an der gleichen Position liegt. "Mensch" und "liegend" kommt dann von der Klassifikation. Genauso können Raumgröße und Position von Türen gelernt werden.

Als Eingangsdaten für die Objektklassifikation dienen die erkannten Objekte mit ihren jeweiligen Punktwolken. Aus der Menge an Raumpunkten die einem Objekt in jedem Verarbeitungszyklus zugeordnet werden, können bestimmte Merkmale abgeleitet werden. Die räumliche Verteilung der Punkte ergibt Länge, Breite, Höhe sowie die Schwerpunktposition des Objekts. Aus den Geschwindigkeitswerten der einzelnen Punkte wird die minimale, maximale und die mittlere Geschwindigkeit ermittelt. Die Merkmale werden in jedem Verarbeitungszyklus ermittelt und nach einer bestimmten Anzahl an Zyklen zu einem Merkmalsvektor zusammengefasst. Dabei werden je nach Anwendung Merkmale kombiniert, gefiltert oder nur bestimmte verwendet. Anhand aufgezeichneter und manuell klassifizierter Daten wird ein Klassifikator trainiert und evaluiert. Dazu wird eine Clusteranalyse aller Merkmalsvektoren im Merkmalsraum durchgeführt. Der trainierte Klassifikator entscheidet anhand der Distanzen zu den ermittelten Clusterschwerpunkten. Es gibt eine Klassifikation die menschlichen Objekte erkennt. Eine weitere Klassifikation ermittelt die Körperhaltung und unterscheidet zwischen Liegen, Sitzen, Stehen und Gehen.

In einer Ausgestaltung des computerimplementierten Verfahrens wird für das phasengesteuerte Antennen-Array eine binäre Phasenmodulation angewendet. Durch die binäre Phasenmodulation können alle Antennen des phasengesteuerten Antennen-Arrays gleichzeitig Senden und Empfangen, wobei die Überlagerung nachfolgen herausgerechnet werden kann, um die Antennensignale einzeln zu verarbeiten. Das gleichzeitige Senden verbessert das Signal-Rausch-Verhältnis und damit die Genauigkeit von Amplitude und Phase.

In einer anderen Ausgestaltung des computerimplementierten Verfahrens wird für eine Überwachung von Objekten in dem Überwachungsbereich eine Zoom-In-Funktion genutzt. Dabei werden die Raumbereiche mit dem erkannten und zu bewertenden Zielobjekt in kleinere Raumbereiche unterteilt, um eine genauere Position des Objektes zu ermitteln.

In einer weiteren Ausgestaltung des computerimplementierten Verfahrens wird für eine Detektion von statischen Objekten in dem Überwachungsbereich eine Kreisapproximation angewendet. Die Kreisapproximation in der komplexen Ebene hat den Vorteil, dass damit statische Objekte besser herausgerechnet werden können als mit der Mittelwertmethode.

In einer anderen weiteren Ausgestaltung des computerimplementierten Verfahrens wird für die Berechnung der Phasenwerte ein erweiterter Differenzierungs- und Kreuzmultiplikationsalgorithmus angewendet. Der erweiterte Differenzierungs- und Kreuzmultiplikationsalgorithmus (Extended DACM) kann anstelle einer acrtan-Funktion zur Berechnung der Phase angewendet werden, dies erlaubt eine schnellere Berechnung der Phasenwerte.

In einer weiteren Ausgestaltung des computerimplementierten Verfahrens wird den Objekten ein Bewegungsindex zugeordnet und die Berechnung der Vitalparameter erfolgt nur bei einer geringen Bewegungsintensität. Erfolgt die Berechnung der Vitalparameter nur bei geringer Bewegungsintensität, hat dies den Vorteil, dass damit verlässlichere Werte für die Bewertung des detektierten Objektes erfasst werden können. Jedoch werden keine Vitalparameter für Objekte in Bewegung ausgewertet.

Der Vorteil des erfindungsgemäßen Verfahrens liegt darin, dass zuverlässig erkannt werden kann, ob es sich bei dem detektierten Objekt um ein Lebewesen, z. B. um einen Menschen oder ein Tier, handelt und ob dieser/dieses gestürzt ist oder nicht und ob dieser/diese Anzeichen einer Erkrankung zeigt oder nicht. Auf Grundlage der ermittelten Vitalparameter, die aus den empfangenen Phasenwerten der Reflexionssignale extrahiert werden, lässt sich ein Gesundheitszustand des Lebewesens ableiten.

Die Aufgabe der Erfindung wird anordnungsseitig ebenfalls durch eine Sensoranordnung gemäß dem unabhängigen Anspruch 14 gelöst. Die erfindungsgemäße Sensoranordnung zur Messung und Überwachung von Vitalparametern und Erkennung von Notfall- und Sturzsituationen umfasst eine Sende- und Empfangseinheit sowie Mittel, die so konfiguriert sind, dass sie die Schritte des Verfahrens nach Anspruch 1 ausführen.

In einer Ausgestaltung der erfindungsgemäßen Sensoranordnung umfasst die Sensoranordnung ein System-on-Chip (SoC), welches ein analoges Frontend, einen digitalen Signalprozessor, einen Mikrocontroller und einen Speicher aufweist, die miteinander kommunizierend über Signalleitungen verbunden sind sowie ein phasengesteuertes Antennen-Array, welches ausgebildet ist, Radarsignale auszusenden und deren Reflexionssignale zu empfangen, und welches durch den Mikrocontroller ansteuerbar ist, wobei die empfangenen Reflexionssignale in dem digitalen Signalprozessor und / oder dem Mikrocontroller verarbeitbar sind.

In einer anderen Ausgestaltung der erfindungsgemäßen Sensoranordnung weist diese ein Thermopile Array zur Detektion einer Temperatur des Objektes im Überwachungsbereich auf.

Die Nutzung des Thermopile Arrays hat den Vorteil, dass das erfasste Wärmebild als Plausibilitätsprüfung der erfassten Reflexionssignale der Radardaten und umgekehrt genutzt werden kann. Die Temperaturmessung kann durch die Abstandsinformationen aus der Radarmessung präzisiert werden, indem Korrekturfaktoren eingeführt werden. Durch die Kombination können beispielsweise lebende Objekte von Sachobjekten unterschieden werden, wobei auch eine Klassifizierung von lebenden Objekten, z. B. Mensch / Tier erfolgen kann.

Dafür wird ein geometrisches Kameramodell durch den Sensor berechnet. Mit Hilfe dieses Modells kann eine direkte Umrechnung von 3D-Raumpunkten des Radars in die 2D-Pixelebene des Thermopile Arrays erfolgen. Eine genaue Zuordnung bestimmter Pixel und damit auch Temperaturen zu einem vom Radar detektierten Objektes erhöht die Genauigkeit der Anwesenheits- und Sturzerkennung. Bewegte Sachobjekte (z. B. kippender Stuhl, Ventilator) werden durch ihre zu geringe Temperatur von der Erkennung ausgeschlossen. Der Sensor berechnet dafür eine Projektionsmatrix, die sich aus der inneren und äußeren Orientierung der Kamera (Thermopile Array) ergibt. Die äußere Orientierung ist die Lage des Sensors im Raum. Die translatorischen Parameter sind über die Konfiguration, d. h. der Positionierung der Sensoranordnung im Raum, bekannt. Die rotatorischen Parameter werden durch die Auswertung eines Beschleunigungssensors ermittelt. Für die innere Orientierung wird das physikalische Lochkameramodell angenommen, welches Brennweite, Pixelgröße und Bildhauptpunkt benötigt. Außerdem wird die Verzerrung der Fischaugenoptik modelliert. Die Verzerrung ist radialsymmetrisch und die Krümmungskurve der Linse ist dem Sensor bekannt. Die Projektionsmatrix wird bei jedem Start des Sensors neu berechnet.

In einer weiteren Ausgestaltung der erfindungsgemäßen Sensoranordnung weist der von der Sensoranordnung erfassbare Raum eine Größe von wenigstens 5m x 5m auf.

Vorteilhaft ist, dass die erfindungsgemäße Sensoranordnung eine kombinierte Sensorik zur Erfassung und Bestimmung von Vitalparametern nutzt, welche eine Sende- und Empfangseinheit für Radarstrahlung und ein Thermopile Array umfasst. Zu den Vitalparametern werden u. a. die Herzschlagrate, die Herzfrequenz, die Atmung bzw. Atemfrequenz, die Körpertemperatur, die Gesichtstemperatur und Bewegungen gezählt. Diese sind mittels der erfindungsgemäßen Sensoranordnung und des computerimplementierten Verfahrens bestimmbar.

Die Auswertung führt zu einer größeren Trennschärfe zwischen gesund und krank des erfassten Lebewesens bzw. der Erkennung eines Notfalls. Sie ermöglicht das Erkennen von Krankheitsbildern und die Differenzierung akuter Notfälle, wie Stürze (mittels Sturzerkennung, Bewegungsanalyse), Herzinfarkt, Sepsis, Lungenembolie und andere. Eine geeignete Kommunikationseinheit realisiert die, bevorzugt drahtlose, Information an die erforderlichen bzw. geeigneten Personen und/oder Institutionen.

Vorteilhaft ist, dass das erfindungsgemäße System kontaktlos und über Distanzen von bis ca. 4 m zuverlässig arbeitet, wobei die Parameter kontinuierlich erfasst werden, d. h. ein kontinuierliches Monitoring der Parameter umgesetzt wird.

Die Erfindung soll nachfolgend an einigen Ausführungsbeispielen näher erläutert werden.

Die Zeichnungen zeigen
- Fig. 1: Schematische Darstellung eines Teilschrittes des erfindungsgemäßen computerimplementierten Verfahrens zur Bestimmung gültiger Reflexionswerte;
- Fig. 2: Zeitlicher Verlauf des Phasensignals der aufgenommenen Atem- und Herzfrequenz bei einer Abtastrate von 20Hz;
- Fig. 3: Vergleich einer durch das erfindungsgemäße Verfahren bestimmten Atemfrequenz und einem am Körper eines Menschen angelegten Referenzmessgerätes;
- Fig. 4: Vergleich einer durch das erfindungsgemäße Verfahren bestimmten Herzschlagrate mittels Radarmessung und einem am Körper eines Menschen angelegten Referenzmessgerätes;
- Fig. 5: Schematische Darstellung des erfindungsgemäßen computerimplementierten Verfahrens in einem Ablaufdiagramm;
- Fig. 6: Schematische Darstellung eines Szenario I: (a) Peron geht durch den Überwachungsbereich und stürzt, (b) Zeitlicher Verlauf der Objekterkennung, (c) Thermopile-Bild während des Sturzes der Person, (d) Visualisierung der Punktwolke vor und nach dem Sturz;
- Fig. 7: Schematische Darstellung eines Szenario II: (a) Peron liegt im Bett, steht auf und stürzt, (b) Zeitlicher Verlauf der Objekterkennung, (c) und (d) aufgenommenes Phasensignal und daraus ermittelte Atem- und Herzschlagrate;
- Fig. 8: Einbindung der Sensoranordnung mit dem erfindungsgemäßen Verfahren in ein zentralisiertes Notrufsystem.

Mit der erfindungsgemäßen Sensoranordnung zur Messung und Überwachung von Vitalparametern und Erkennung von Notfall- und Sturzsituationen, die eine Sende- und Empfangseinheit sowie Mittel umfasst, die so konfiguriert sind, dass sie die Schritte des erfindungsgemäßen computerimplementierten Verfahrens ausführen kann, können Lebewesen/Objekte in einem Überwachungsbereich detektiert werden.

Die Sensoranordnung mit integrierter Auswertung ist verwendbar für die Durchführung von Bio-Scan und/oder Bio-Monitoring mittels Messung von Einzelvariablen und deren Evaluierung zur integrierten Betrachtung in einem lernenden System, d. h.
- Kontaktlose kombinierte Messung von Herzfrequenz, Atemfrequenz, Körpertemperatur und Bewegungen,
- Lernendes System mittels integrierendem Algorithmus zur Erhöhung der Trennschärfe zwischen krank und gesund im Vergleich zur Betrachtung der Einzelparameter,
- Erweiterbare/anpassbare Evaluierung durch Änderung und/oder Ergänzung der Algorithmen an verschiedenste Krankheitsbilder und/oder Integration weiterer Messungen.

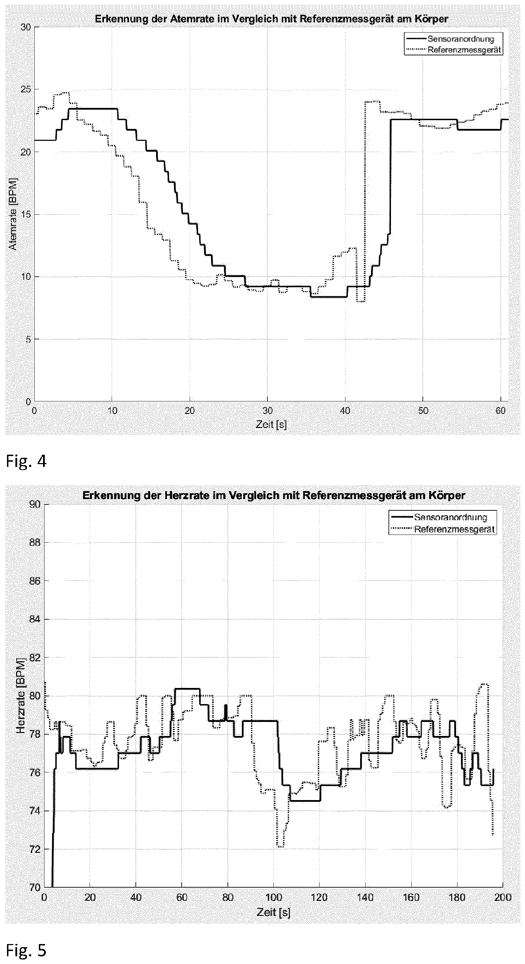

Figur 3 zeigt den Vergleich einer durch das erfindungsgemäße Verfahren bestimmten Atemfrequenz und einem am Körper eines Menschen angelegten Referenzmessgerätes bzw. Figur 4 zeigt den Vergleich einer durch das erfindungsgemäße Verfahren bestimmten Herzschlagrate mittels Radarmessung und einem am Körper eines Menschen angelegten Referenzmessgerätes. Die Vergleiche mit den Referenzmessgeräten zeigen, dass die durch die Sensoranordnung aufgenommenen und mit dem Verfahren verarbeiteten Daten hinreichend genau sind, um daraus Rückschlüsse beispielsweise auf den Gesundheitszustand eines Menschen ziehen zu können.

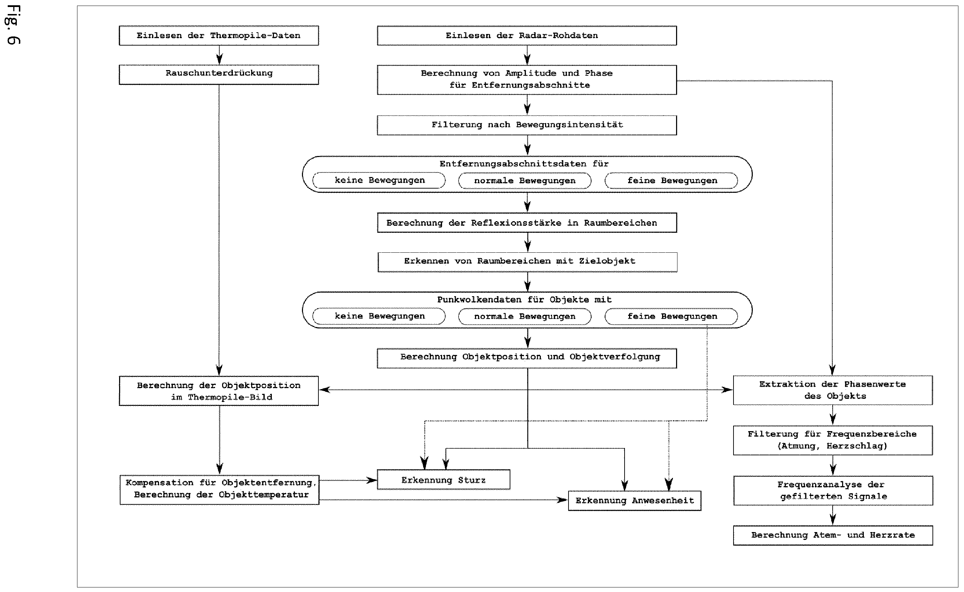

Figur 5 zeigt das erfindungsgemäße computerimplementierte Verfahren im Überblick, wie es in der vorliegenden Anmeldung detailliert beschrieben ist.

Für die Ermittlung der Anwesenheits-/Sturz- und Vitalparametererkennung werden von einer Sendeeinheit 2 Radarsignale im 60-64 GHz Bereich transmittiert, die im Überwachungsbereich 1 an Hindernissen/Objekten 3 reflektiert werden und von einer Empfangseinheit wieder empfangen werden. Aus den empfangenen Reflexionssignalen werden Entfernungsdaten berechnet, wobei für jede 3D-Position im Überwachungsbereich aus den empfangenen Reflexionsdaten eine Reflexionsstärke berechnet wird. Mittels einer Plausibilitätsprüfung werden die gültigen Reflexionswerte aus der Menge der berechneten Reflexionswerte ermittelt und diese zu einer Punktwolke zusammengefasst. Einzelne Punkte der Punktwolke werden anschließend zu einem Objekt gruppiert und über eine Objektklassifikation klassifiziert. Die Detektion und Überwachung des Objektes erfolgt anhand der X-/Y-Position im Überwachungsbereich, wobei durch eine zusätzliche sowohl räumliche als auch zeitliche Überwachung einer Z-Position des Objektes detektiert werden kann, ob ein Sturzereignis eingetreten ist oder nicht. Durch eine Messung der Vitalparameter des Objektes, die aus Phasenwerten der aufgenommenen Reflexionssignale für dieses Objekt ermittelt und ausgewertet werden und mit deren Verknüpfung mit den Werten der Anwesenheits- und Sturzerkennung kann eine Plausibilitätsprüfung durchgeführt werden, ob das Objekt ein Lebewesen ist, welches gestürzt ist oder ob nur ein Sachobjekt z. B. eine Vase, heruntergefallen ist. Wenn es sich tatsächlich um ein Lebewesen handelt, können auch Rückschlüsse auf den Gesundheitszustand des Lebewesens geschlossen werden.

In einem besonderen Ausführungsbeispiel kann das erfindungsgemäße Verfahren in der Tiermedizin eingesetzt werden, beispielsweise für die Überwachung von Pferden in einer Stallbox. Ein Pferd, welches zu Koliken neigt oder bei dem ein Verdacht auf eine Erkrankung besteht oder welches fohlt, kann über die erfindungsgemäße Sensoranordnung überwacht werden. Aus den Bewegungsdaten und den Vitalparameter des Pferdes, die erfasst und miteinander verknüpft werden, lassen sich Rückschlüsse auf den Gesundheitszustand des Pferdes ziehen. Auch können aus den Daten Krankheitsbilder abgeleitet werden, um eine erste Diagnostik durchzuführen. Dies erleichtert die Arbeit von Tierärzten und entlastet auch die Tierhalter, die nicht ständig vor Ort sein müssen.

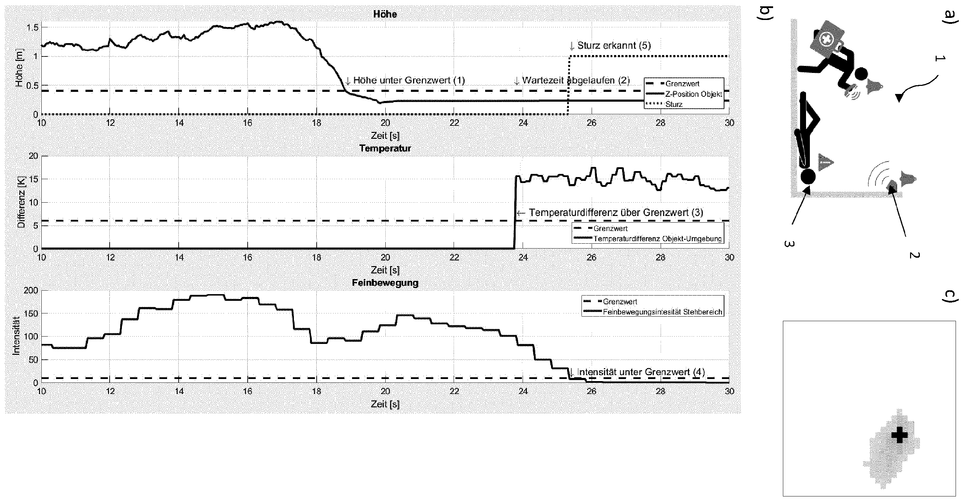

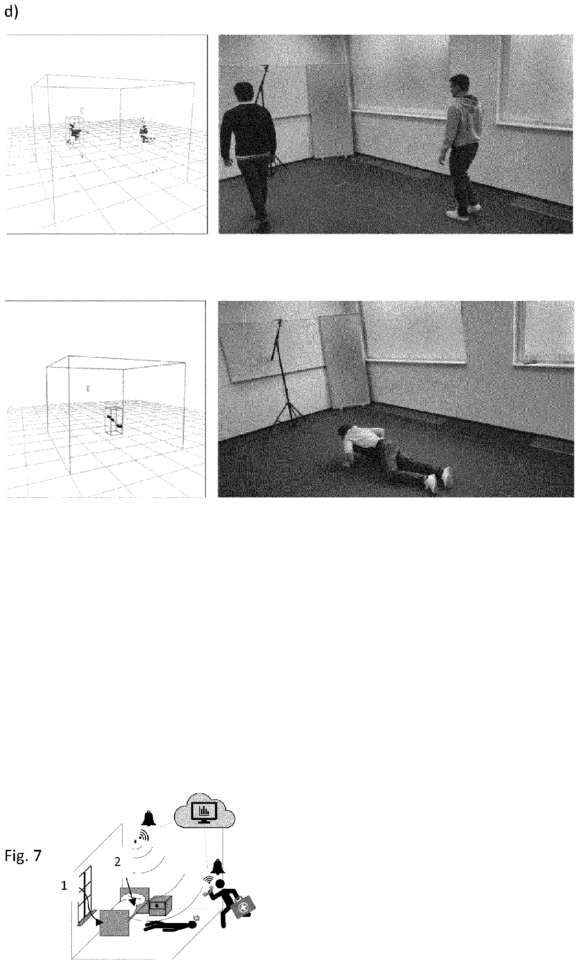

Figur 6 zeigt ein klassisches Anwendungsszenario für das erfindungsgemäße computerimplementierte Verfahren und die dazugehörige Sensoranordnung: Eine Person geht durch einen Raum und stürzt (Fig. 6a). Mit der Sensoranordnung werden die Bewegungen der Person/des Objektes zeitlich aufgenommen. Das erfindungsgemäße Verfahren durchläuft dabei folgende zeitliche Schritte, die für eine Sturzerkennung erfüllt sein müssen (Fig. 6b):
1. Die Z-Position eines Objekts ist kleiner als ein Grenzwert;
2. Die Z-Position bleibt für eine bestimmte Zeit unter einem Grenzwert;
3. Berechnung und Überprüfung der Temperaturdifferenz des Objekts zur Umgebung, Differenz ist größer als Grenzwert;
4. Intensität der Feinbewegung über einer bestimmten Höhe ist kleiner als ein Grenzwert;
5. Sturz erkannt, Aussenden einer Alarmmeldung.

Fig. 6c) zeigt das Thermopile-Bild während eines Sturzes. Graue Pixel entstehen durch die Körperwärme des am Boden liegenden Menschen. Das schwarze Pixelkreuz wurde vom Radarchip in das Bild eingefügt und ist die 3D-Position umgerechnet in die 2D-Pixelkoordinaten. Anhand dieser Koordinaten wird die Temperaturdifferenz zwischen Objekt und der unmittelbaren Umgebung berechnet.

Fig. 6d) zeigt zum einen die realistische, von der Sensoranordnung zu detektierende Szene zum anderen die Visualisierung der Punktwolke des detektierten Objekts/der detektierten Objekte vor und nach dem Sturz.

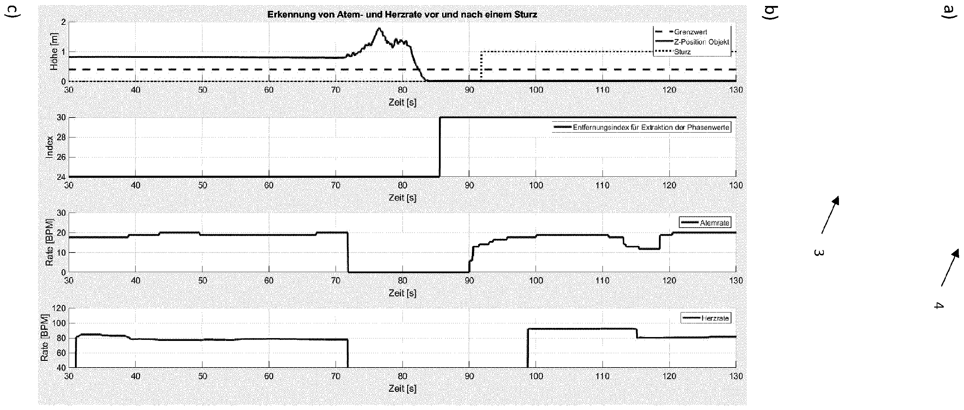

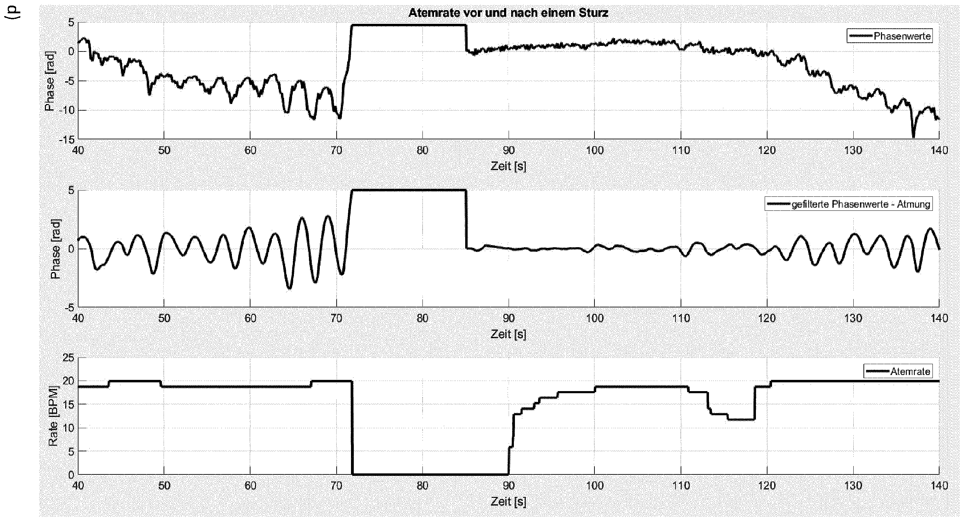

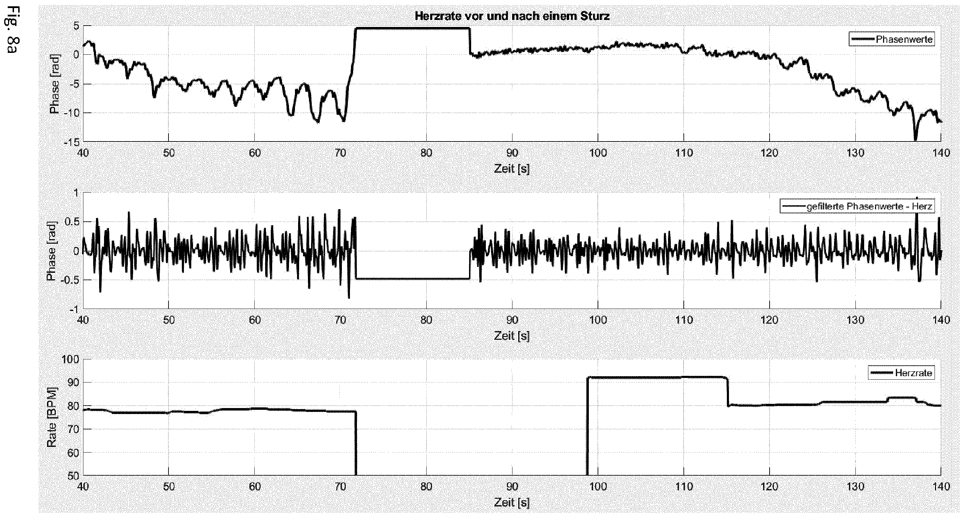

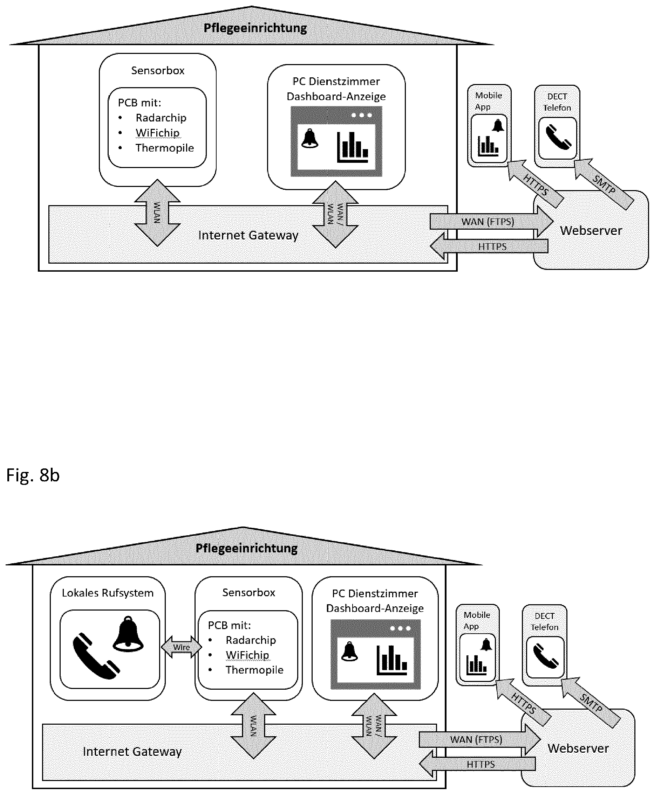

Figur 7 zeigt ein weiteres klassisches Anwendungsszenario für das erfindungsgemäße computerimplementierte Verfahren und die dazugehörige Sensoranordnung: Eine Person liegt im Bett, steht auf und stürzt. Im Bett werden kontinuierlich Atem- und Herzschlagrate erfasst (Fig. 7b). Bei einer Änderung der Objektposition wird die Ermittlung direkt auf die neue Position umgestellt. Die Phasenextraktion stellt dem Algorithmus sofort die neuen Phasensignale aller virtuellen Antennen zur Verfügung. Dadurch werden kurz nach der Sturzmeldung wieder kontinuierlich Werte für die Vitalparameter übertragen. Fig. 7c) und 7d) zeigen das Phasensignal mit einer kurzen Unterbrechung und den aus dem Phasensignal ermittelten Atem- und Herzschlagraten vor und nach dem Sturz (Unterbrechung). Figur 8 zeigt die Einbindung der Sensoranordnung 2 mit dem erfindungsgemäßen Verfahren in ein zentralisiertes Notrufsystem, a) ohne und b) mit einem lokalen Notrufsystem.

Zusammenfassend kann die Sensoranordnung mit dem erfindungsgemäßen computerimplementieren Verfahren zur Erkennung von Notfall- und Sturzsituationen und zur Bestimmung von Vitalparametern beispielsweise für den Notruf, die Telemedizin, die kontaktlose Diagnose aus der Distanz, das Gesundheits-Monitoring verwendet werden. Dazu zählen beispielsweise:
- Bio-Scan mittels kontaktlosem Screening von Personen auf infektiöse Risiken.
- Screening des Gesundheitszustandes von Personen bei Zugang zu kritischen Infrastrukturen und gesundheitlich geschwächten Personen, z.B. in Intensivbehandlung befindlichen Personen oder nach Organtransplantationen oder in vergleichbaren Situationen, durch Messing von Vitalparametersignalen, wie Herzfrequenz, Atemfrequenz, Gesichtstemperatur. Die integrierte Interpretation der Messungen erhöht die Trennschärfe des Tests.
- Gesundheits-Monitoring zum kontaktlosen Messen der Vitalparameter von Personen, beispielsweise der Herz- und Atemfrequenz und der Temperatur, in Verbindung mit einer Bewegungsanalyse zur Erfassung von Stürzen u. ä.

Die kontaktlose Messung und die Auswertung von Vitalparametern wie Atemfrequenz, Pulsfrequenz, Temperatur, Bewegung gestatten verschiedene Anwendungen, wie
- Automatisierter Notruf
- Screening auf infektiöse Risiken, beispielsweise für Zugangskontrollen in öffentlichen Einrichtungen, Behörden, Flughäfen, Firmen, Infektionshotspot, zu Risikogruppen oder für die Vorauswahl für Tests,
- Monitoring von Vitalparameteren, beispielsweise zu folgenden Zwecken:
   a) Gesundheits-Monitoring,
   b) Meldung von Grenzüberschreitungen,
   c) Erkennen von Notfällen, wie bei Herzinfarkt, Embolie, Schlafapnoe u. a.
   d) Messung aus der Distanz im Bereich von 0,3 m bis 5 m, beispielsweise durch Anordnung der Sensoranordnung an einer Raumdecke (Abstand ca. 1 - 2 m),
- Telemedizin mit elektronischem Wartezimmer, beispielsweise einschließlich Meldung von Grenzwertüberschreitungen, und Ferndiagnostik,
- Bewegungserkennung zur Sturzerkennung und/oder Smart Home Anwendungen,
- Tiermedizin, beispielsweise ebenfalls mittels Monitoring und/oder Diagnose.

### Bezugszeichenliste

- 1: Überwachungsbereich
- 2: Sende- und Empfangseinheit
- 3: zu detektierendes Objekt
- 4: Verarbeitungs- und Auswerteeinheit

## Patentansprüche

1. Computerimplementiertes Verfahren zur Messung und Überwachung von Vitalparametern sowie Erkennung von Notfall- und Sturzsituationen in einem Überwachungsbereich (1), umfassend die folgenden Schritte:
- Empfangen von Daten, die mittels einer Sende- und Empfangseinheit (2) erfasst werden, wobei die Sende- und Empfangseinheit (2) ausgebildet ist Radarsignale in den Überwachungsbereich (1) auszusenden und ihre Reflexionssignale aus dem Überwachungsbereich (1) zu empfangen,
- Berechnen von Entfernungsdaten aus den empfangen Reflexionssignalen,
- Berechnen einer Reflexionsstärke für jede 3D-Position im Überwachungsbereich (1) aus den empfangenen Reflexionssignalen,
- Ermitteln gültiger Reflexionswerte aus der Reflexionsstärke für jede 3D-Position im Überwachungsbereich (1) und Erstellen einer Punktwolke,
- Gruppieren einzelner Punkte der Punktwolke zu einem Objekt (3) und Verfolgen einer Position des Objektes (3) im Überwachungsbereich (1), wobei
- für eine Anwesenheitserkennung eines Objektes (3) eine X- / Y-Position im Überwachungsbereich (1) detektiert und überwacht wird, und
- für eine Sturzerkennung eines Objektes (3) zusätzlich zu einer X- / Y-Position im Überwachungsbereich (1) sowohl eine räumliche als auch zeitliche Überwachung einer Z-Position des Objektes (3) detektiert und überwacht wird, und
- für eine Messung der Vitalparameter eines Objektes (3) Phasenwerte der aufgenommenen Reflexionssignale dieses Objektes (3) ausgewertet und mit den Werten der Anwesenheits- und Sturzerkennung verknüpft werden.

2. Computerimplementiertes Verfahren nach Anspruch 1, wobei mittels einer Wärmesignatur an einer Objektposition, die mittels eines Temperatursensors erfasst wird, Rückschlüsse auf die Präsenz des Objektes (3) geschlossen werden.

3. Computerimplementiertes Verfahren nach Anspruch 1 oder 2, wobei mittels einer Feinbewegungsprüfung auf Basis von Phasenwerten, die für jedes gruppierte Objekt (3) aus den empfangenen Reflexionssignalen für jede 3D-Position im Überwachungsbereich (1) extrahiert werden, detektiert wird, ob sich das Objekt (3) bewegt oder nicht.

4. Computerimplementiertes Verfahren nach Anspruch 3, wobei die Feinsteuerungsprüfung die folgenden Schritte zur Bestimmung von Vitalparametern, insbesondere einer Atemfrequenz umfasst:
- Auswählen einer Objektposition und der dazugehörigen Reflexionssignale sowie Phasenwerte in einem Raumbereich,
- Filtern der Reflexionssignale mit einem IIR-Bandpassfilter für den Atemfrequenzbereich,
- Durchführen einer Frequenzanalyse mittels Fourier-Transformation und Berechnen eines Amplitudenspektrums für den Atemfrequenzbereich,
- Ermitteln der höchsten Amplitudenwerte des Amplitudenspektrums sowie deren entsprechende Frequenzwerte und Grundrauschen,
- Sortieren der ermittelten Amplitudenwerte nach ihrem Signal-Rausch-Verhältnis, wobei ein vorbestimmter Prozentsatz der ermittelten Amplitudenwerte zur Weiterverarbeitung ausgewählt und verwendet wird,
- Berechnen eines Medianwertes aller Frequenzwerte der ausgewählten und verwendeten Amplitudenwerte, wobei der Medianwert einen Wert für die Atemfrequenz darstellt.

5. Computerimplementiertes Verfahren nach Anspruch 3, wobei die Feinbewegungsprüfung die folgenden Schritte zur Bestimmung von Vitalparametern, insbesondere einer Herzschlagrate umfasst:
- Auswählen einer Objektposition und der dazugehörigen Reflexionssignale sowie Phasenwerte in einem Raumbereich,
- Filtern der Reflexionssignale mit einem IIR-Bandpassfilter für den Frequenzbereich der Herzschlagrate,
- Durchführen einer Frequenzanalyse mittels Fourier-Transformation und Berechnen eines Amplitudenspektrums für den Frequenzbereich der Herzschlagrate,
- Bereinigen des berechneten Amplitudenspektrums von Störungen durch harmonische Frequenzen der Atemfrequenz und zufälliger Objektbewegungen, indem harmonische Frequenzen der potentiellen Herzschlagrate detektiert und ein Fundamentalfrequenzwert in einem Datenpuffer gewichtet gespeichert wird,
- Durchführen einer Clusteranalyse auf die in dem Datenpuffer gespeicherten Frequenzen, wobei ein Frequenzmittelwert des größten Clusters gespeichert wird,
- Wiederholen des vorbenannten Verarbeitungsschrittes und Berechnen eines Medianwertes der über mehreren Verarbeitungszyklen gespeicherten Frequenzmittelwerte, wobei der Medianwert einen Wert für die Herzschlagrate darstellt.

6. Computerimplementiertes Verfahren nach Anspruch 5, wobei bei der Bereinigung des berechneten Amplitudenspektrums nur Frequenzwerte in den Datenpuffer übernommen werden, die folgende Regeln erfüllen:
- Ist eine 1. und 2. Harmonische des ausgewählten Frequenzwertes vorhanden, wird der Frequenzwert viermal in den Datenpuffer übernommen,
- Ist die 1. oder 2. Harmonische des ausgewählten Frequenzwertes vorhanden, wird der Frequenzwert dreimal in den Datenpuffer übernommen,
- Ist der Amplitudenwert der höchste im Amplitudenspektrum und ist der zugehörige Frequenzwert keine Harmonische der Atemfrequenz wird der Frequenzwert zweimal übernommen,
- Ist eine Differenz zwischen einem Frequenzwert zu einem der drei höchsten Amplitudenwerte im Amplitudenspektrum und einem Ergebnis für die Herzschlagrate aus einem vorherigen Verarbeitungszyklus kleiner als ein Grenzwert, wird dieser Frequenzwert übernommen, wobei
- die Regeln in der vorbenannten Reihenfolge überprüft werden bis eine Regel zutrifft.

7. Computerimplementiertes Verfahren nach einem der vorherigen Ansprüche, wobei ein Vertrauenslevel definiert wird, welches genutzt wird, um zu prüfen, ob berechnete Werte der Vitalparameter plausibel sind oder ob es sich um falsch positiv detektierte Vitalparameter handelt.

8. Computerimplementiertes Verfahren nach einem der vorherigen Ansprüche, wobei der Überwachungsbereich (1) konfiguriert wird, indem Objekte (3) mittels einer Objektklassifikation klassifiziert werden unddie Daten der Objektklassifikation für einen selbstlernenden Algorithmus für das Erstellen und kontinuierliche Verbessern einer Raumkonfiguration des Überwachungsbereiches genutzt wird.

9. Computerimplementiertes Verfahren nach einem der vorherigen Ansprüche, wobei für das phasengesteuerte Antennen-Array eine binäre Phasenmodulation angewendet wird.

10. Computerimplementiertes Verfahren nach einem der vorherigen Ansprüche, wobei für eine Überwachung von Objekten in dem Überwachungsbereich eine Zoom-In-Funktion genutzt wird.

11. Computerimplementiertes Verfahren nach einem der vorherigen Ansprüche, wobei für eine Detektion von statischen Objekten (3) in dem Überwachungsbereich (1) eine Kreisapproximation angewendet wird.

12. Computerimplementiertes Verfahren nach einem der vorherigen Ansprüche, wobei für die Berechnung der Phasenwerte ein erweiterter Differenzierungs- und Kreuzmultiplikationsalgorithmus angewendet wird.

13. Computerimplementiertes Verfahren nach einem der vorherigen Ansprüche, wobei den Objekten (3) ein Bewegungsindex zugeordnet wird und die Berechnung der Vitalparameter nur bei einer geringen Bewegungsintensität erfolgt.

14. Sensoranordnung zur Messung und Überwachung von Vitalparametern und Erkennung von Notfall- und Sturzsituationen mit einer Sende- und Empfangseinheit (2) sowie Mitteln (4), die so konfiguriert sind, dass sie die Schritte des Verfahrens nach Anspruch 1 ausführen.

15. Sensoranordnung nach Anspruch 14, wobei die Sensoranordnung ein System-on-Chip umfasst, welches ein analoges Frontend, einen digitalen Signalprozessor, einen Mikrocontroller und einen Speicher aufweist, die miteinander kommunizierend über Signalleitungen verbunden sind sowie ein phasengesteuertes Antennen-Array, das durch den Mikrocontroller ansteuerbar ist, wobei die empfangenen Daten durch die Sensoranordnung in dem digitalen Signalprozessor und / oder dem Mikrocontroller der Sensoranordnung weiter verarbeitbar sind.

16. Sensoranordnung nach einem der vorherigen Ansprüche, wobei die Sensoranordnung ein Thermopile Array zur Detektion einer Temperatur eines Objektes im Überwachungsbereich (1) aufweist.

17. Sensoranordnung nach einem der vorherigen Ansprüche, wobei der von der Sensoranordnung erfassbare Raum eine Größe von wenigstens 5m x 5m aufweist.
